Europäisches Patentamt

European Patent Office

Office européen des brevets

(11) Veröffentlichungsnummer: **0 224 500**
**B1**

(12) **EUROPÄISCHE PATENTSCHRIFT**

(45) Veröffentlichungstag der Patentschrift:
06.12.89

(21) Anmeldenummer: 86902786.2

(22) Anmeldetag: 23.05.86

(86) Internationale Anmeldenummer:
PCT/CH 86/00067

(87) Internationale Veröffentlichungsnummer:
WO 86/06952 (04.12.86 Gazette 86/26)

(51) Int. Cl.⁴: **A 61 B 17/08**

(54) **WUNDKLAMMER.**

(30) Priorität: 31.05.85 CH 2307/85

(43) Veröffentlichungstag der Anmeldung:
10.06.87 Patentblatt 87/24

(45) Bekanntmachung des Hinweises auf die Patenterteilung:
06.12.89 Patentblatt 89/49

(84) Benannte Vertragsstaaten:
AT BE CH DE FR GB IT LI LU NL SE

(56) Entgegenhaltungen:
FR-A-419 096
FR-A-709 422
FR-A-1 324 556
GB-A-456 458
US-A-1 644 625
US-A-3 385 299
US-A-3 601 127

(73) Patentinhaber: WALDER- UTZ, Alice, Dr.,
Obstgartenstrasse 31, CH- 8006 Zürich (CH)

(72) Erfinder: WALDER- UTZ, Alice, Dr.,
Obstgartenstrasse 31, CH- 8006 Zürich (CH)

(74) Vertreter: Feldmann, Clarence Paul, c/o
Patentanwaltsbüro FELDMANN AG Postfach
Kanalstrasse 17, CH- 8152 Glattbrugg (CH)

**Beschreibung**

Nach jeder chirurgischen Operation kommt der Wundheilung grosse Bedeutung zu. Man ist daher bestrebt, optimale Voraussetzungen für eine möglichst risikolose, schmerzfreie und schnelle Heilung zu schaffen. Es ist bekannt, dass Wunden, deren Schnittränder durch Klammern oder Fäden zusammengehalten werden, schneller und kosmetisch schöner heilen als Wunden, die man der Selbstheilung überlässt (Williams & Harisson 1977). In vielen Teilbereichen der Medizin hat deshalb die Klammertechnik neben der Nahttechnik ihren festen Platz, besonders bei gynäkologischen und Abdominaloperationen. Neuerdings hat die Klammertechnik vermehrt wieder in anderen Bereichen Anwendung gefunden. Sie bietet im Vergleich zur Nahttechnik viele technische und medizinische Vorteile. Bei ihrer Anwendung ist die Infektionsrate niedriger, Granulome treten dabei nicht auf und die durchschnittliche Spitalaufenthaltsdauer des Patienten ist daher kürzer (Beresford et al. 1984). Bei Hautverbrennungen wird das Klammern deshalb vorgezogen, weil es viel schneller ist als das Anbringen einer Fadennaht. Das Hauttransplantat kann schneller plaziert werden und die kritische Anästhesiezeit kann daher verringert werden (Kahn et al. 1984, Hallock et al. 1984). Stephens und Niesche (1974) empfehlen die Hautklammerung für Wundverschlüsse bei Abdominaloperationen, Halsoperationen, Brust-und Thoraxoperationen, Nockemann (1968) für Hautverschlüsse nach Schilddrüsenoperationen. Nach Swanson (1982) eignet sich die Klammerung an der Kopfhaut, am Gesicht und an den Extremitäten. Durch die im Vergleich zur Fadennaht reduzierte Traumatisation des Gewebes zeigt die Klammerung eine schönere Wundheilung und eine geringe Narbenbildung. Nach Nockemann (1965/1968) hinterlassen Nähte an den Einstichstellen, sowie an den Stellen, an denen der Faden eine zeitlang das Gewebe einschnürte oder drückte, eine Narbe. Diese Narben können besonders an der Haut oft recht störend und unschön wirken. Man hat deshalb versucht, die Einstichstellen nach Zahl und Grösse möglichst klein zu halten und die narbigen Drucklinien, die vom Faden verursacht werden und das bekannte Strickleiter-Syndrom erzeugen, gänzlich zu vermeiden.

Diese Bestrebungen führten zum Wundverschluss der Haut mittels Klammern. Ihre Applikation und Entfernung ist einfach und schnell. Zudem ist die Gefahr der Keimansiedlung auf dem Klemmenmetall gering, weil auf seiner glatten Oberfläche die Keime schlecht haften und Metall ausserdem eine eigene, keimtötende bakterizide Wirkung hat. Im Gegensatz dazu erhöht die Verwendung von Nähmaterial in der Wunde das Risiko der Virulenz der Staphylokokken um einige 10000 mal. Die Abszessrate ist bei der Klammertechnik etwa dreimal niedriger. Nach Stephens (1970) zeigen mit Klammern verschlossene Wunden am 7. postoperativen Tagbessere mechanische Eigenschaften als mit einer Fadennaht verschlossene, dass heisst, der Elastizitätsmodul ist grösser, die Zugfestigkeit höher und die Fähigkeit zur Energieabsorption ohne Ruptur besser. Lowdon (et al. 1982) fand auch, dass die postoperativen Komplikationen in Form von Anastomosenschwäche, Wundinfektion und Blutungen bei Verwendungen von Klammern um rund 25 % seltener sind. Ein Versuch von Meiring (et al. 1982) an je 20 Patienten zeigte auch, dass der Wundverschluss durch Klammern um etwa 80 % schneller und zudem einfacher durchgeführt werden konnte als mit einer konventionellen Fadennaht.

Diese vielen offensichtlichen Vorteile zeigen die grundsätzliche Bedeutung der Klammertechnik, die sich vor allem in den USA schon in den meisten Spitälern gegen die Nahttechnik durchgesetzt hat. Trotzdem haben die heute gebräuchlichen Klammern noch entscheidende Mängel, die eliminiert werden müssen.

Herkömmliche Klammern für die Applikation an allen Körperteilen, mit Ausnahme der Kopfhaut, durchstechen ausnahmslos die beiden zusammenzuhaltenden Hautseiten der Wunde an mehreren Stellen. Zum einen ist dieses Durchstechen für den Patienten schmerzhaft und zum anderen aus medizinischen Gründen ungünstig. Neben der kosmetischen Beeinträchtigung durch die Stiche erhöhen diese vor allem die Infektionsgefahr, da Fremdmaterial in die Wunde eindringt. Zudem erfordert die Entfernung der Klammern bei vielen Klammersystemen ein spezielles Gerät, gestaltet sich nicht einfach und ist daher zeitaufwendig.

Im Fachhandel ist bereist eine Hautklammer bekannt, welche die zusammenzuhaltenden Hautteile nicht durchsticht. Diese Klammer wird nur in der Neurochirurgie zur Trepanation gebraucht. Sie wird nur temporär eingesetzt, dass heisst nur zum groben Abklemmen der Gefässe während der Operation, da ja in der Neurochirurgie massive Blutverluste in Kauf genommen werden müssen. Dieses Abklemmen quetscht und traumatisiert die Haut sehr stark, aber der Blutverlust kann so eingedämmt werden. Sie kann aus obengenannten Gründen nur *während* und nicht nach der Operation angewendet werden, wie beispielsweise zum Wundverschluss. Sie ist von ihrer Konstruktion her ausschliesslich an der Kopfhaut applizierbar, da sie nur eine sehr geringe Spreizung erlaubt und deshalb eben nur dort angewendet werden kann, wo die Unterhautschicht sehr dünn ist und direkt auf dem Knochen (Schädel) aufliegt.

Ausserdem muss diese Hautklammer für das Anlegen wie für das Abnehmen mit einer speziellen Zange bedient werden.

Die Druckkanten dieser Kopfhautklammer sind wellenförmig gestaltet, wobei immer ein Wellenhügel gegenüber einem Wellental zu liegen kommt. Das verursacht Zug- und Druckkräfte auf die Haut, die in verschiedenen Richtungen wirken und bei längerer Belassung der Klammern narbige Spuren hinterlassen können. Diese Klammer unterscheidet sich von der erfingungsgemässen

Wundklammer also nicht nur durch den Anwendungsbereich, durch die Funktion und durch ihre Zielsetzung, sondern auch durch ihre Konstruktion.

Aufgabe der vorliegenden Erfindung ist es, die genannten Nachteile herkömmlicher Klammern unter Beibehaltung der grundsätzlichen Vorteile der Klammertechnik zu eliminieren und eine Hautklammer zu schaffen, die an allen Körperteilen einfach und ohne Durchstechung der Haupt appliziert werden kann.

Erfindungsgemäss wird diese Aufgabe gelöst durch eine Merkmalen des Patentanspruches 1. Bevorzugte Ausführungen der Wundklammer werden in den abhängigen Ansprüchen definiert. Beispielhafte Ausführungen der erfindungsgemässen Wundklammer werden in den nachfolgenden Zeichnungen der Deutlichkeit halber vergrössert dargestellt. Es zeigen:

Figur 1 eine perspektivische Ansicht einer Wundklammer aus zwei gegeneinander schwenkbaren Teilen.

Figur 2 vorteilhafte Ausbildungen der Druckkanten der Klammerteile,

Figur 3 eine perspektivische Ansicht von zwei parallel verschiebbaren Klammerteilen,

Figur 4 eine Frontansicht einer aus den Wundklammerteilen der Figur 3 zusammengesetzten Klammer mit Blattfeder,

Figur 5 die entsprechende Klammer mit Druckfeder und

Figur 6 mit Zugfeder.

Figur 7 eine Wundklammer mit Schubstangen.

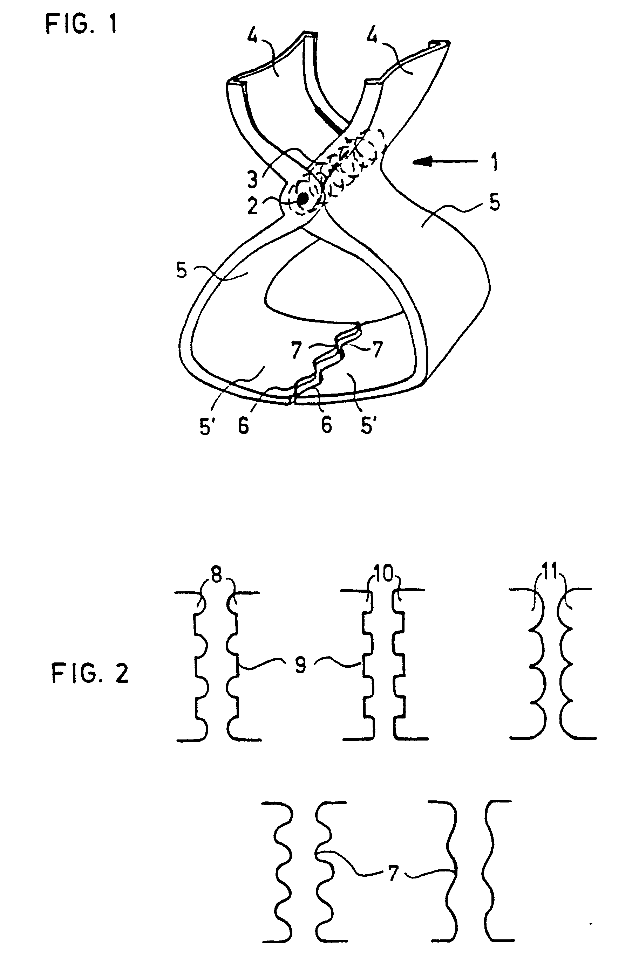

Das in der Figur 1 gezeigte Ausführungsbeispiel der Wundklammer ist nach dem Wäscheklammer-Prinzip konstruiert, dass heisst, die beiden Klammerteile 1 sind gegeneinander schwenkbar auf einer gemeinsamen Achse 2 angeordnet. Eine Torsionsfeder 3, die um die Achse 2 angelegt ist, drückt die als Griffe 4 ausgebildeten Seiten der Klammerteile 1 auseinander und deshalb die Klemmschenkel 5 entsprechend gegeneinander. Diese Konstruktion erlaubt durch die Anwendung des Hebelgesetzes eine grosse Spreizung der Klemmschenkel 5 bei vergleichsweise kleiner Schwenkung der Griffe 4. Ferner sind die Griffe 4 mit bloss zwei Fingern zu fassen und die Klammer ist daher einfach zu bedienen. Die Druckkanten 6 der Klemmschenkel 5, die dazu bestimmt sind, mit der Haut in Berührung zu kommen, sind in diesem Ausführungsbeispiel wellenförmig ausgebildet. Die Wellenkuppen 7 der einander gegenüberliegenden Druckkanten liegen zueinander in Opposition. Die Rundungen der Wellenkuppen verhindern einerseits ein Durchstechen der Haut und andererseits erhöhen sie den spezifischen Druck auf die Haut und verbessern damit die Haftung der applizierten Klammer im Vergleich zu geraden Druckkanten. Ein weiterer Vorteil dieser Ausführung besteht darin, dass die Klammer für postoperative Schwellungen im Nahtbereich der Wunde oberhalb der Druckkanten genügend Raum lässt. Es gibt Klammersysteme, bei denen das nicht der Fall ist und welche in der Narbe deshalb Spuren hinterlassen, die unter dem Namen "Strickleiter-Syndrom" bekannt sind. Die Klemmschenkel 5 verlaufen von der Achse 2 erst in einer geschwungenen Linie nach aussen. Im untersten Bereich sind die Klemmschenkel um ungefähr 90° zu einander abgewinkelt, so dass die Klemmschenkelflächen 5' der Klemmschenkel etwa in einer Ebene liegen. Diese Ebene berührt nunmehr die Haut etwa tangential. Die Druckkanten 6 der Klemmchenkelflächen 5' schieben somit die Wundränder annähernd parallel zur Hautoberfläche zusammen.

In Figur 2 sind weitere Beispiele von vorteilhaften Druckkanten 6 an den Klemmschenkeln 5 gezeigt. Alle diese Ausbildungen sind so ausgelegt, dass sie die Haut niemals durchstechen und trotzdem eine genügende Haftung der Klammer auf der Haut gewährleisten, wobei höchstens die Epithelschicht der Haut traumatisiert wird.

Figur 2a) zeigt Druckkanten mit einander gegenüberliegenden, halbkreisförmigen Kuppen 8, die voneinander einen gewissen Abstand 9 haben. In Figur 2b) haben die Druckkanten kurze, gerade Erhebungen 10 mit abgerundeten Ecken, die ebenfalls mit einem gewissen Abstand 9 voneinander angeordnet sind. In Figur 2c) werden die Erhebungen durch Kreissegmente 11 gebildet, die arkadenförmig aneinandergereiht sind. Die Ausführungen in Figur 2d) und 2e) zeigen Erhebungen, die durch Wellenkuppen 7 von Wellen mit unterschiedlichen Krümmungsradien gebildet werden.

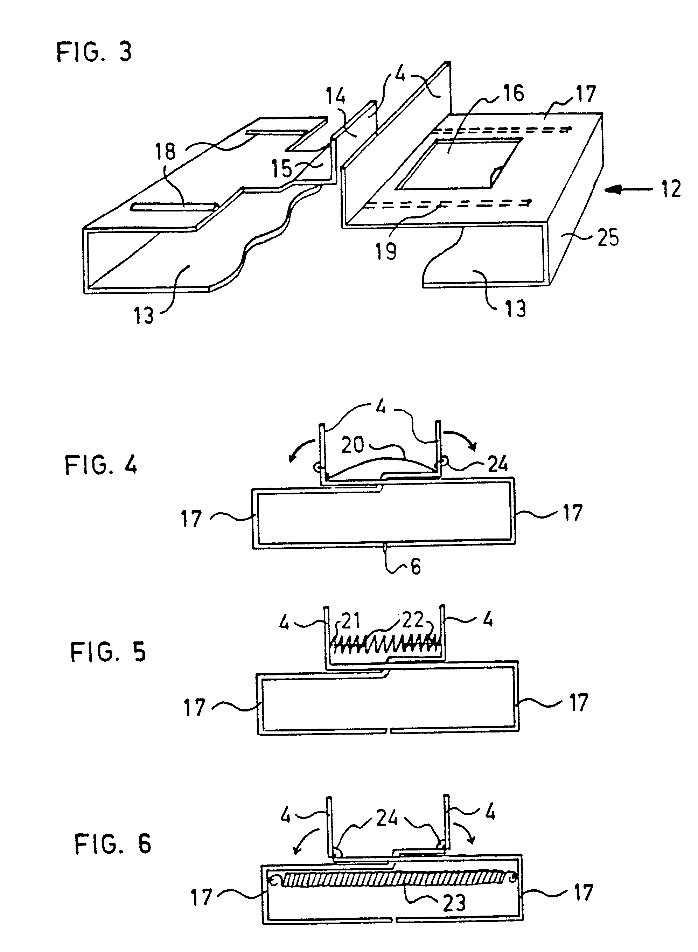

In Figur 3 sind zwei Wundklammerteile 12 für eine besonders flache Wundklammer in perspektivischer Ansicht zu sehen. Beide Teile bestehen aus gestanzten Plättchen, aus denen durch zweimaliges, rechtwinkliges Abkanten in der gleichen Richtung Klemmflächen 13 geformt sind, während durch je ein rechtwinkliges Abkanten in der anderen Richtung am anderen Ende der Wundklammerteile 12 Griffe senkrecht zu den Klemmflächen 13 geschaffen sind. Der eine Griff 14 ist dabei schmäler und als Lasche ausgebildet, welche durch eine Versetzung ihres Fusses 15 um eine Metallplättchendicke nach oben in ein entsprechendes Fenster 16 im anderen Klammerteil 17 passt, wo sie Halt findet, wenn die Führungen 18 bei der zusammengesetzten Klammer in die entsprechenden Nuten 19 eingelegt sind. Werden nun die Griffe 4 gegeneinander gedrückt, so verschieben sich die Klemmflächen in einer Ebene parallel auseinander.

Für das Schliessen der beiden Klemmflächen flächen sorgt gemäss der Ausführung nach Figur 4 eine zwischen den Griffen 4 eingelegte Blattfeder 20, welche diese Griffe 4 auseinandergedrückt und damit die Druckkanten 6 gegeneinander presst. In Figur 5 wird diese Aufgabe von einer Druckfeder 21 übernommen, die an beiden Griffen 4 auf Stiften 22 geführt ist, die zueinander fluchtend verlaufen und etwa 1/3 der Länge der Druckfeder aufweisen. Das Zusammenpressen der Druckkanten 6 kann auch mittels einer zwischen den Klammerteilen 17 eingehängten Zugfeder 23 erreicht werden (Fig.6).

Die Griffe 4 in Figur 4 und Figur 6 sind ausserdem mit Scharnieren 24 versehen, die ein Hinunterklappen der Griffe 4 nach erfolgter Applikation der Wundklammer ermöglichen. Die Bauform gemäss den Figuren 3 - 6 ermöglicht eine besonders flache Ausführung. Die Seitenwände 25 der rechteckigen Klammerteile 12 müssen genügend hoch sein, um einer eventuellen Wundschwellung Platz zu bieten. Sind die Griffe 4 zudem noch klappbar gestaltet, so ist die Gesamthöhe der Klammer im Verhältnis zur Breite besonders günstig. Dies ist von Bedeutung, wenn über die Klammern ein Verband angelegt wird, wird damit doch der Druck auf die Klammer verringert und es besteht auch kaum noch eine Kippgefahr durch Kräfte, die vom Verband auf die Klammer ausgeübt werden. Gleichzeitig wird der spezifische Anpressdruck auch noch verringert durch die Grösse der Klemmflächen 13, welche der applizierten Wundklammer zusätzlich auch Stabilität geben. Die parallele Führung der ammer teile 17 verhilft dazu, dass die Wunde nur parallel zur näheren Hautoberfläche zusammengedrückt wird, was eine geringe Narbenbildung begünstigt.

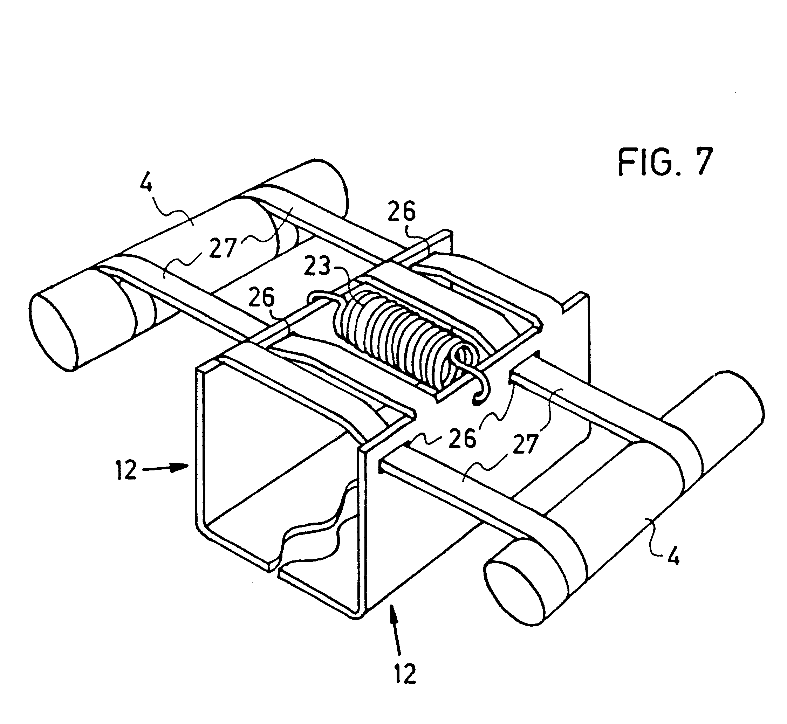

In Figur 7 ist eine besonders vorteilhafte Ausführung der Erfindung gezeigt. Die Klammerteile 12 haben parallel zur Klemmvorrichtung je zwei Schubstangen 27 angeformt, welche je beim gegeüberliegenden Klammerteil durch ein Fenster 26 geführt sind. Diese insgesamt vier Fenster 26 vermitteln den vier Schubstangen 27 eine Führung und gewährleisten damit, dass beim Zusammendrücken der an ihnen befestigten Griffe 4 die Klammerteile 12 parallel voneinander weggeschoben werden. Die Griffe 4 bestehen hier aus Rundstählen, um welche die Enden der Schubstangen 27 in einer dazu vorgesehenen Nut gebogen sind. Bei auseinandergedrückten Klammerteilen 12 wird die zwischen diesen Teilen 12 eingehängte Zugfeder 23 gestreckt. Das Applizieren der Wundklammer erfolgt dann durch das Loslassen der Griffe 4, wobei die beiden Klammerteile 12 kraft der Zugfeder 23 gegeneinander gedrückt werden.

Die einfache Form ermöglicht eine preiswerte Herstellung und Montage der Klammer. Auch diese neuen Wundklammern können aus inertem, rostfreiem Stahl gefertigt werden. Eine Verwendung anderer Materialien wie zum Beispiel Kunststoffe ist nicht ausgeschlossen.

Mit der Tatsache, dass diese neue Hautklammer die Haut an keiner Stelle mehr durchsticht, sind viele Vorteile verbunden. Zum ersten werden die Schmerzen für den Patienten erheblich reduziert; zweitens wird ein kosmetisch besseres Resultat erreicht, denn es treten keine bleibende Einstichstellen oder Vernarbungen von Einstichstellen mehr auf. Aus medizinischer Sicht ist weiter die bedeutend geringere Infektionsgefahr wichtig, was dadurch erreicht wird, dass Fremdmaterial nunmehr bloss mit der Hauptoberfläche und nicht mehr mit dem Gewebe und der Wunde in Berührung kommt. Als weiterer Vorteil gegenüber verschiedenen herkömmlichen Klammersystemen ist zu erwähnen, dass durch die Konstruktion der neuen Hautklammer für das Gewebe bei eventueller postoperativer Schwellung Platz zum Ausweichen bleibt. Weil die erfindungsgemässe Wundklammer sehr weit gespreizt werden kann, ist eine Applikation an allen Körperteilen möglich. Das Anlegen wie das Entfernen erfolgt mit bloss zwei Fingern und ohne Hilfsinstrument.

## Patentansprüche

1. Wundklammer für das Zusammenhalten von Wundrändern während dem Heilungsprozess, bestehend aus zwei relativ zueinander beweglichen Klammerteilen (1, 12) mit je einem Griff (4), die unter Federkraft in Schliessrichtung zusammengedrückt werden, wobei die Klammerteile (1, 12) je eine, annähernd in einer Ebene verlaufende, zueinander gerichtete Klemmfläche (5', 13) aufweisen, die jeweils in gleiche wellenlinienförmige Druckkanten (6) mit gerundeten Wellenkuppen (7, 8, 10, 11) auslaufen, wobei die Erhebungen beider Druckkanten (6) einander gegenüberliegen.

2. Wundklammer nach Anspruch 1, dadurch gekennzeichnet, dass die beiden gegeneinander beweglichen Klammerteile (1) auf einer gemeinsamen Achse (2) schwenkbar gelagert sind, wobei um die Achse eine Torsionsfeder (3) angeordnet ist.

3. Wundklammer nach Anspruch 1 dadurch gekennzeichnet, dass die beiden Klammerteile (12) aneinander und relativ zueinander in einer Ebene parallel verschiebbar geführt sind und Kraft der Feder gegeneinander gedrückt werden.

4. Wundklammer nach Anspruch 3, dadurch gekennzeichnet dass die beiden Klammerteile (12) je zwei parallel angeordnete Schubstangen (27) angeformt haben, die je beim gegenüberliegenden Wundklammerteil durch ein Fenster (26) geführt sind und an deren Enden Griffe (4) angebracht sind und dass die Feder eine Zugfeder (23) ist.

5. Wundklammer nach Anspruch 3, dadurch gekennzeichnet, dass die Feder eine Blattfeder (20) ist, die zwischen die beiden Griffe (4) eingespannt ist und diese auseinanderpresst.

6. Wundklammer nach Anspruch 3, dadurch gekennzeichnet, dass die Feder eine Zugfeder (23) ist, welche die beiden Klammerteile (12) gegeneinanderzieht.

7. Wundklammer nach Anspruch 3, dadurch gekennzeichnet dass die Feder eine Druckfeder (21) ist, die zwischen den Griffen (4) der Wundklammer eingespannt ist und auf jeder Seite auf einem Stift (22) gehalten ist.

8. Wundklammer nach Anspruch 3, dadurch gekennzeichnet dass die als Griffe (4) ausgebildeten Klammerteile mittels Scharnieren (24) abklappbar sind.

9. Wundklammer nach Anspruch 1 dadurch gekennzeichnet dass die beiden Klammerteile (12) durch mindestens ein Arretiermittel in Schliessstellung so gehalten sind, dass die wellenlinienförmigen Druckkanten einen freien Spalt definieren.

10. Wundklammer nach Anspruch 9, dadurch gekennzeichnet, dass das Arretiermittel biegbar und damit die Spaltbreite einstellbar ist.

## Claims

1. Wound clips for holding together the edges of a wound during the healing process, consisting of two clip components (1, 12) movable relative to each other and each having a grip (4), the components being sprung so as to be pressed together in the closing direction, each clip component (1, 12) having one clipping surface (5', 13) directed towards each other and running approximately in a single plane, each terminating in undulating compression edges (6) with rounded peaks (7, 8, 10, 11), the peaks of the two compression edges (6) lying opposite each other.

2. Wound clip according to claim 1, characterised in that the two clip components (1) which can be displaced relative to each other pivot on a common axis (2) with a torsion spring (3) being disposed around the axis.

3. Wound clip according to claim 1, characterised in that the two clip components (12) can be displaced towards each other and relatively to each other in parallel in a single plane and are pressed against each other by the force of the spring.

4. Wound clip according to claim 3, characterised in that both clip components (12) have two push bars (27) arranged in parallel, each bar being led through an aperture in the opposite clip component and grips (4) being fitted to the ends of the bars and that the spring is a tension spring (23).

5. Wound clip according to claim 3, characterised in that the spring is a leaf spring (20) which is fixed between the two grips (4) and pushes these apart.

6. Wound clip according to claim 3, characterised in that the spring is a tension spring (3), which pulls the two clip components (12) against each other.

7. Wound clip according to claim 3, characterised in that the spring is a compression spring (21), which is fixed between the grips (4) of the wound clip and held on each side on a pin (22).

8. Wound clip according to claim 3, characterised in that the two clip components designed as grips (4) can be folded down by means of hinges (24).

9. Wound clip according to claim 1, characterised in that the two clip components (12) are held in the closed position by at least one stop device so that the compression edges which are undulating in shape define a free space.

10. Wound clip according to claim 9, characterised in that the stop device is flexible and the width of the gap is therefore adjustable.

## Revendications

1. Agrafe chirurgicale pour maintenir ensemble les lèvres d'une plaie pendant le processus de guérison, comprenant deux parties (1, 12) qui sont mobiles l'une par rapport à l'autre, possèdent une poignée respective (4) et sont poussées conjointement dans le sens de la fermeture sous la force d'un ressort, ces parties (1, 12) de l'agrafe présentant une surface respective de pincement (5', 13) s'étendant approximativement dans un plan, tournée vers son homologue et s'achevant, à chaque fois, par des bords presseurs identiques (6) de forme ondulée à lobes ondulés arrondis (7, 8, 10, 11), les protubérances des deux bords presseurs (6) se faisant mutuellement face.

2. Agrafe chirurgicale selon la revendication 1, caractérisée par le fait que les deux parties (1) de cette agrafe, mobiles l'une par rapport à l'autre, sont montées à pivotement sur un axe commun (2), un ressort de torsion (3) étant enroulé autour de cet axe.

3. Agrafe chirurgicale selon la revendication 1, caractérisée par le fait que les deux parties (12) de cette agrafe sont guidées à coulissement parallèle dans un plan, l'une sur l'autre et l'une par rapport à l'autre, et sont poussées l'une contre l'autre par la force du ressort.

4. Agrafe chirurgicale selon la revendication 3, caractérisée par le fait que les deux parties (12) de cette agrafe présentent deux tiges respectives de poussée (27) ménagées solidairement et disposées parallèlement, qui sont guidées à travers une fenêtre (26) sur la partie opposée considérée de l'agrafe chirurgicale, et aux extrémités desquelles des poignées (4) sont façonnées; et par le fait que le ressort est un ressort de traction (23).

5. Agrafe chirurgicale selon la revendication 3, caractérisée par le fait que le ressort est une lame de ressort (20) qui est bandée entre les deux poignées (4), et repousse ces dernières à l'écart l'une de l'autre.

6. Agrafe chirurgicale selon la revendication 3, caractérisée par le fait que le ressort est un ressort de traction (23), qui tire l'une contre l'autre les deux parties (12) de l'agrafe.

7. Agrafe chirurgicale selon la revendication 3, caractérisée par le fait que le ressort est un ressort de pression (21) qui est bandé entre les poignées (4) de l'agrafe chirurgicale, et est retenu de part et d'autre sur une broche (22).

8. Agrafe chirurgicale selon la revendication 3, caractérisée par le fait que les parties de cette agrafe, réalisées sous la forme de poignées (4), sont rabattables au moyen de charnières (24).

9. Agrafe chirurgicale selon la revendication 1, caractérisée par le fait que les deux parties (12) de cette agrafe sont retenues en position fermée par l'intermédiaire d'au moins un moyen d'arrêt, de telle sorte que les bords presseurs de forme ondulée délimitent un interstice libre.

10. Agrafe chirurgicale selon la revendication 9, caractérisée par le fait que le moyen d'arrêt est flexible, de sorte que la largeur de l'interstice est réglable.

FIG. 1

FIG. 2

FIG. 3

FIG. 4

FIG. 5

FIG. 6

FIG. 7